# EUROPEAN PATENT APPLICATION

(11) **EP 1 479 692 A1**
(43) Date of publication of application: **24.11.2004**
(21) Application number: 04077160.2
(22) Date of filing: 01.08.1997
(51) Int. Cl.: C07K 14/245, C07K 7/08, A61K 38/08, A61P 31/04, A61K 38/10

(54) **Peptides responsive to antibodies against a consensus peptide of the CS4-CFA/I family proteins**

(30) Priority: 02.08.1996 US 23076; 05.08.1996 US 23145
(62) Divisional of application: 97936322.3
(71) Applicant: Department of the Army, U.S. Government U.S. Army Medical Research & Materiel Command, Elizabeth Arwine, Fort Detrick, MD 21702-5012 (US)
(72) Inventor: Cassels, Frederick, Ellicott City MD 21043 (US); Loomis-Price, Lawrence, North Bethesda MD 10852 (US)
(74) Representative: Williams, Aylsa

(57) **Abstract**

This invention relates to amino acid sequences from within a consensus peptide of the formula: VEKNITVTASVDPTIDLLQADGSALPSAVALTYSPA (Seq. #1). Eight mer peptides from within the consensus peptide were tested against an antibody raised to the consensus peptide. Studies relating to antibody raised to denatured proteins from the natural organisms producing the family of proteins were also useful and showed particular value of some sequences. A sequence of the formula ASVDPTIDLLQA (Seq. #2) was identified thereby. An enlarged sequence of the formula TVTASVDPTIDLLQAD (Seq. #3) is also especially interesting as are intermediate sequences such as sequences VTASVDPTIDLLQAD (Seq. #4), TASVDPTIDLLQAD (Seq. #5), and TASVDPTIDLLQA (Seq. #6) as being binding sites for antibodies raised to the denatured proteins.

## Description

### Field of the Invention:

This invention relates to amino acid sequences from within a consensus peptide of the formula:
VEKNITVTASVDPTIDLLQADGSALPSAVALTYSPA (Seq.#1) Eight mer peptides from within the consensus peptide were tested against an antibody raised to the consensus peptide. Studies relating to antibody raised to denatured proteins from the natural organisms producing the family of proteins was also useful and showed particular value of some sequences. A sequence of the formula ASVDPTIDLLQA (Seq, #2) was identified thereby. An enlarged sequence of the formula TVTASVDPTIDLLQAD (Seq. #3) is also especially interesting as are intermediate sequences such as sequences VTASVDPTIDLLQAD (Seq. #4), TASVDPTIDLLQAD (Seq. #5), and TASVDPTIDLLQA (Seq. #6) as being binding sites for antibodies raised to the denatured proteins.

### Background of the invention:

The effect of E. coli in mammals is dependent on the particular strain of organism. Many beneficial E. coli are present in the intestines. Since the initial association with diarrheal illness, five categories of diarrheagenic E. coli have been identified and are presently recognized: enterotoxigenic (ETEC), enteropathogenic (EPEC), enterohemorrhagic (EHEC), enteroaggregative (EAggEC), and enteroinvasive (EIEC). These categories are grouped according to characteristic virulence properties, such as elaboration of toxins and colonization factors and/or by specific types of interactions with intestinal epithelial cells. ETEC are the most common of the diarrheagenic E. coli and pose the greatest risk to travelers. E. coli of the family CS4-CFA/I are some of the more common enterotoxigenic E. coli. There is need for vaccines which are specific against this class of E. coli that give rise to antibodies that cross-react with and cross-protect against the more common members of the CS4-CFA/I family. There are six members of this family of ETEC fimbrial proteins, CFA/I, CS1, CS2, CS4, CS17 and PCF 0166. ETEC are responsible for high infant mortality in developing countries, with an estimate that almost 800,000 deaths per year due to these organisms. These organisms also cause illness in adult travelers to regions where the disease is endemic.

Colonization factor antigens (CFA) of ETEC are important in the initial step of colonization and adherence of the bacterium to intestinal epithelia. In epidemiological studies of adults and children with diarrhea, CFA/I is found in a large percentage of morbidity attributed to ETEC. The CFA/I is present on the surfaces of bacteria in the form of pili (fimbriae), which are rigid, 7 nm diameter protein fibers composed of repeating pilin subunits. The CFA/I antigens promote mannose-resistant attachment to human brush borders with an apparent sialic acid sensitivity.

A study of proteins in E. coli belonging to the CS4-CFA/I family resulted in the finding that the N-terminal region of the protein maintains a high degree of sequence identity between members of this group. Immunological evidence shows that cross-reaction exists between members of the family CS4-CFA/I.

Cassels, et al. have identified a consensus peptide of 36 amino acids which acts as an immunogen raising antibodies against the proteins of all members of the E. coli family CS4-CFA/I. The region of the protein represented in the subunit encompasses known linear B- and T-cell epitopes of CFA/I. The consensus peptide has a high level of homology to strains bearing six different colonization factors. The consensus peptide is of the formula:

VEKNITVTASVDPTIDLLQADGSALPSAVALTYSPA (Seq. #1)

### Description of the Invention:

It is the purpose of this invention to identify specific epitopes that may be used to give rise to antibodies which will agglutinate all members of the E. coli family CS4-CFA/I. It is a further purpose of this invention to identify subunits of the consensus peptide previously identified by Cassels which will act as immunogens for purposes of raising antibodies against the CS4-CFA/I family proteins.

It is, furthermore, a purpose of this invention to provide kits for use in identifying all members of the CS4-CFA/I family of E. coli and antibodies to such organisms in clinical and environmental samples. The antibodies were raised to the consensus peptide of Sequence #1.

### Materials and Methods.

### Peptide synthesis:

Blocks of pins for cleavable syntheses were obtained from Chiron Mimotopes U.S. Peptide synthesis was carried out according to the manufacturer's instructions using Opfp-derivatized amino acids. Peptides of length 8 with 7 overlap were manufactured in order to locate all linear epitopes in the sequence with a highest redundancy. Peptides had a linker-the amino acids Ser -Gly-Ser-Gly-- and biotin covalently coupled to the N-terminus, for a total length of 12 amino acids (17 reactions, including the biotin). Peptides were cleaved from the pins using 0.1 M phosphate buffer, pH 8.0, containing 40% acetonitrile. Two peptides were made and sacrificed for amino acid analysis as proof of peptide purity. A dinitrophenol (DNP) pin was included so that the efficiency of the cleavage could be monitored spectrophotometrically.

A total of 29 peptides encompassing the entire 36 amino acid consensus peptide were synthesized as follows:

1.  VEKNITVT (Seq. #7)  16.  DLLQADGS (Seq. #22)
2.  EKNITVTA (Seq. #8)  17.  LLQADGSA (Seq. #23)
3.  KNITVTAS (Seq. #9)  18.  LQADGSAL (Seq. #24)
4.  NITVTASV (Seq. #10)  19.  QADGSALP (Seq. #25)
5.  ITVTASVD (Seq. #11)  20.  ADGSALPS (Seq. #26)
6.  TVTASVDP (Seq. #12)  21.  DGSALPSA (Seq. #27)
7.  VTASVDPT (Seq. #13)  22.  GSALPSAV (Seq. #28)
8.  TASVDPTI (Seq. #14)  23.  SALPSAVA (Seq. #29)
9.  ASVDPTID (Seq. #15)  24.  ALPSAVAL (Seq. #30)
10.  SVDPTIDL (Seq. #16)  25.  LPSAVALT (Seq. #31)
11.  VDPTIDLL (Seq. #17)  26.  PSAVALTY (Seq. #32)
12.  DPTIDLLQ (Seq. #18)  27.  SAVALTYS (Seq. #33)
13.  PTIDLLQA (Seq. #19)  28.  AVALTYSP (Seq. #34)
14.  TIDLLQAD (Seq. #20)  29.  VALTYSPA (Seq. #35)
15.  IDLLQADG (Seq. #21)

Using antibodies had been found to agglutinate E coli having the CS4-CFA/I family proteins, an attempt was made to identify the binding site of response to the 36 mer consensus peptide. One monoclonal antibody raised to the consensus peptide was found to bind with all E. coli of the CS4-CFA/I family.

### ELISA Method:

### Materials:

For blocking, a composition containing 5% nonfat dry milk in PBS + 0.2% sodium azide was used. Stock streptavidin (Calbiochem Corp., LaJolla, CA) at 1 mg/ml in water was kept frozen in aliquots for up to several months. On the day of use, the stock streptavidin was diluted in phosphate buffered saline (PBS) to provide a concentration of 5 µg/ml streptavidin. Goat F(ab')2 anti-mouse, anti-rabbit and anti-human sera were labeled with alkaline phosphatase (Biosource, International, Camarillo, CA).

Streptavidin was plated at 5 µg/ml, 50 µl per well, and incubated over night at 4°C. Plates were then washed by hand (Nunc Immunowash 12 hand plate washer, Fisher Scientific, Pittsburgh, PA) three times with PBS/0.1% Tween 20. Peptides were then diluted to 10 µg/ml in PBS and plated at 50 µl/well. After incubation for one hour at room temperature followed by washing, the peptides were incubated with sera diluted in blocker at appropriate concentrations (5% nonfat dry milk) for 2 hours at room temperature. After washing the wells, 50 µl of phosphatase-labeled anti-serum IgG diluted 1:1000 in blocker was added to each well and was allowed to incubate at room temperature for 1 hour. The plates were washed. The 100 µl of PNDP (p-nitrophenylphosphate) substrate, prepared according to the manufacturer's instructions, was added to each well. Results were read at 5, 15 and 60 minutes using a microtiter plate reader (UVmaxTM, Moelcular Devices, Sunnyvale, CA). Immunization with consensus peptide:
The consensus peptide was conjugated to bovine serum albumin (BSA) or tetanus toxoid followed by conjugation to Streptococcus pneumoniae type 14 polysaccharide. When the
peptide was conjugated to as indicated below, a cysteine was added at the terminal end of the peptide to provide the peptide CVEKNITVTASVDPTIDLLQADGSALPSAVALTYSPA (Seq. #37) The albumin or toxoid was then iodoacetylated. The peptide was mixed with the acetylated albumin or toxoid. (Sulfide bonds are thereby formed between cysteine residues providing a conjugated protein.)

Immunogenic compositions contained complete Freund's adjuvant and were administered to rabbits subcutaneously on day 1. On day 21, a booster shot was given, and on day 32, the animals were bled.

### Example 1:

Rabbits were bled, then immunized on day 0 with a composition containing 280 µg peptide/BSA conjugate in Freund's complete adjuvant. On day 21, the animals were boosted with 140 µg peptide/BSA conjugate in Freund's incomplete adjuvant. Blood was drawn on day 32. The interaction of antibodies raised against the specific antigens of the denatured proteins of the various strains was studied by comparing interaction of serum from the animals obtained on day 0 with response on to serum from the animals obtained on day 32 by Western blot. In all instances, the Western blot was negative for reaction with serum obtained on day 0. The Western blot data on interaction of immune serum collected on day 32 with the denatured proteins is given below with 0 being no reaction and 4 being a strong reaction:

| Titer | 1:50 | 1:500 | 1:5000 | 1:50000 |
|---|---|---|---|---|
| CS1 | 4 | 4 | 4 | 4 |
| CS2 | 4 | 4 | 4 | |
| CS4 | 4 | 4 | 3 | 2 |
| CS17 | 4 | 3 | 2 | 0.5 |
| 0166 | 4 | 1 | 3 | |
| CFA/1 | 4 | 3 | 2 | |

### Example 2:

An immunogenic composition is prepared containing 2800 µg/ml of a conjugate of a peptide of the formula:
VEKNITVTASVDPTIDLLQADGSALPSAVALTYSPA bound to BSA through a cysteine in complete Freunds reagent.

### Example 3:

A immunogenic composition is prepared containing 4000 µg/ml of a peptide of the formula: VEKNITVTASVDPTIDLLQADGSALPSAVALTYSPA in complete Freunds adjuvant.

### Example 4:

Rabbits were given a composition containing 400 µg peptide of the formula:
VEKNITVTASVDPTIDLLQADGSALPSAVALTYSPA in complete Freunds adjuvant. The response was evaluated as in Example 2:

| Titer | 1:50 | 1:500 | 1:5000 | 1:50000 |
|---|---|---|---|---|
| CS1 | 4 | 4 | 2 | 1 |
| CS2 | | | | |
| CS4 | 2 | 0 | 0 | 0 |
| CS17 | 2 | 0 | 0 | 0 |
| 0166 | 4 | 2 | | |
| CFA/I | | | | |

### Example 5:

The same study was done comparing antibodies raised to denatured proteins of PCF 0166.

| Titer | 1:1000 | 1:10000 | 1:100000 |
|---|---|---|---|
| CS1 | 3 | 0.5 | 0 |
| CS2 | 2 | 1 | 0 |
| CS4 | 2 | 0.5 | 0.5 |
| CS17 | 3 | 0.5 | 0 |
| 0166 | 4 | 3 | 1 |
| CFA/I | 3 | 0.5 | 0 |

### Example 6:

Effect of antibody raised to whole CS2 protein was studied in the manner of example 5.

| Titer | 1:1000 | 1:10000 | 1:100000 |
|---|---|---|---|
| CS1 | 4 | 3 | 0.5 |
| CS2 | 2 | 2 | 0 |
| CS4 | 3 | 1 | 0 |
| CS17 | 3 | 1 | 0 |
| 0166 | 4 | 1 | 0 |
| CFA/I | 3 | 0 | 0 |

### Example 7:

Studies were conducted to determine whether antibodies raised to the peptide would cause agglutination of whole bacteria of various strains. Antibody responses to three preparations of consensus peptide antigen were used to immunize the rabbits were compared: 1) peptide conjugated to bovine serum albumin (aPepBS), 2) free peptide (aPepFr) and 3) peptide conjugated to tetanus toxoid and S. pneumonia T14 (aPepTT). The tetanus toxoid was conjugated to the peptide using the described above for conjugation to BSA. The three preparations were used to immunize two animals each. The serum was then contacted with whole bacteria and the slides were inspected for agglutination of the bacteria.

| CF | aPepBSA | aPepFr | aPepTT |
|---|---|---|---|
| CS1 | 1/2 | 0/2 | 1/2 |
| CS2 | 2/2 | 0/2 | 2/2 |
| CS4 | 0/2 | 0/2 | 0/2 |
| CS17 | 0/2 | 0/2 | 0/2 |
| 0166 | 1/2 | 0/2 | 2/2 |
| CFA/1 | 1/2 | 0/2 | 2/2 |

In view of the test data, it is seen that the data indicates that consensus proteins can give rise to antibodies that react to denatured protein and cause agglutination of more than one strain of E. coli of the CS4-CFA/I family. However, it is also seen that conjugation to a larger molecule provides improved properties to the peptides for purposes of raising antibodies to the whole bacteria and the proteins of these organisms.

Antibodies from the rabbits were then tested against the specific 8-mer peptides obtained from Seq. #1 in the manner disclosed above to determine the binding sites of the antibodies in the sera with the consensus peptide.

It appeared, under this method of testing, that the most reactive peptides are those containing peptides 2, 3, 4, 5, 8, 9, 10, 11, 12, 13, 14, 23, 24, 25, 26, 27, 28, and 29 of the formulas:
EKNITVTA (Seq. #8), KNITVTAS (Seq. #9), NITVTASV (Seq #10),
ITVTASVD (Seq. #11), TASVDPTI (Seq. #14), ASVDPTID (Seq. #15), SVDPTIDL (Seq. #16), VDPTIDLL (Seq. #17), DPTIDLLQ (Seq. #18), PTIDLLQA (Seq. #19), SALPSAVA (Seq. #29), ALPSAVAL (Seq. #30), LPSAVALT (Seq. # 31), PSAVALTY (Seq. #32), SAVALTYS (Seq. #33), AVALTYSP (Seq. #34),and VALTYSPA (Seq. #35). In view of this data that epitopes containing these peptides would be preferred for use in reaction with antibodies raised to the consensus peptide. Epitopes containing the peptides ASVDPTID (Seq. #15), SVDPTIDL (Seq. #16), VDPTIDLL (Seq. #17), DPTIDLLQ (Seq. #18), PTIDLLQA (Seq. #19), PSAVALTY (Seq. #32), SAVALTYS (Seq. #33), and AVALTYSP (Seq. #34) were more reliably interactive with the antibodies raised to the consensus peptide. It is also likely that the addition of a proline to either one or both ends of any peptide which does not end with that amino acid would be expected to increase binding ability. The peptide of the formula SAVALTYS (Seq. #33), especially when bounded by a proline to provide PSAVALTYSP (Seq. #36) is a preferred peptide.

### Application of PEPESCAN data to 8 mer units:

Using this method, other sequences including that of the formula ASVDPTIDLLQA (Seq, #2) were identified. An enlarged sequence of the formula TVTASVDPTIDLLQAD (Seq. #3) is also especially interesting as are intermediate sequences such as sequences VTASVDPTIDLLQAD (Seq. #4), TASVDPTIDLLQAD (Seq. #5), and TASVDPTIDLLQA (Seq. #6) as being binding sites for antibodies raised to the denatured proteins.

### Procedure for obtaining antibody to denature subunits:

Partially to fully purified colonization factor (40% to 100% pure) was run on SDS-PAGE gel (5-15 µg/lane of 10 comb gel (precast 10 comb, 1mm thickness, Tris-tricene gel from Novex, San Diego, California), for primary immunization run 9 lanes for each rabbit (45-135 µg CF protein with 1/2 the amount used in the primary immunization used as a booster).

The gel was stained with 0.5% Coomassie Blue (BioRad, Richmond, California) in water for 1 hour, then destained with multiple changes of water for 60-90 minutes. The colonization factor bands were excised with a scalpel and the excess gel trimmed. The bands were stored at -20°C until use.

### Immunization:

After removal from freezer, the gel slices were transferred to a glass tissue homogenizer using a teflon pestle with grooves at the tip. The slices were ground with 0.3 ml phosphate buffered saline (PBS). The drill was run to homogenize the gel for 30 to 45 seconds. (In the instant case, a pestle with a shaft of steel was used which allowed placement of the pestle into the chuck of the hand-held drill.)

The homogenate was transferred to 16 mm X 150 mm test tubes with disposable plastic transfer pipet. The pestle and homogenizer vessel was rinsed repeatedly with PBS and the contents transferred to the 16 x 150 test tube until a volume of 1.2 ml was obtained.

The sample obtained above was placed in a vortex mixer and vortexed on high. Freund's adjuvant 1.2 ml was added. (Complete Freund's was used for the primary immunization, while incomplete Freund's was used for the boost.) The composition was vortexed until a thick emulsion of almost a butter consistency was obtained (12-20 minutes).

a preimmune serum was obtained on all animals, which were then immunized with the 1 ml of the emulsion subcutaneously at 4-6 spots on the shoulders and rump. The animals were boosted three weeks later, then bleed 10 days after the booster shots.

The 8-mer peptides were exposed to the serum of the animals and the samples examined by means disclosed above to determine whether binding had occurred. The data is shown below.

Testing with antibodies indicated those epitopes which bound to the antibodies. As a result, it was possible to identify those epitopes which were most likely to bind to antibodies in a serum sample.

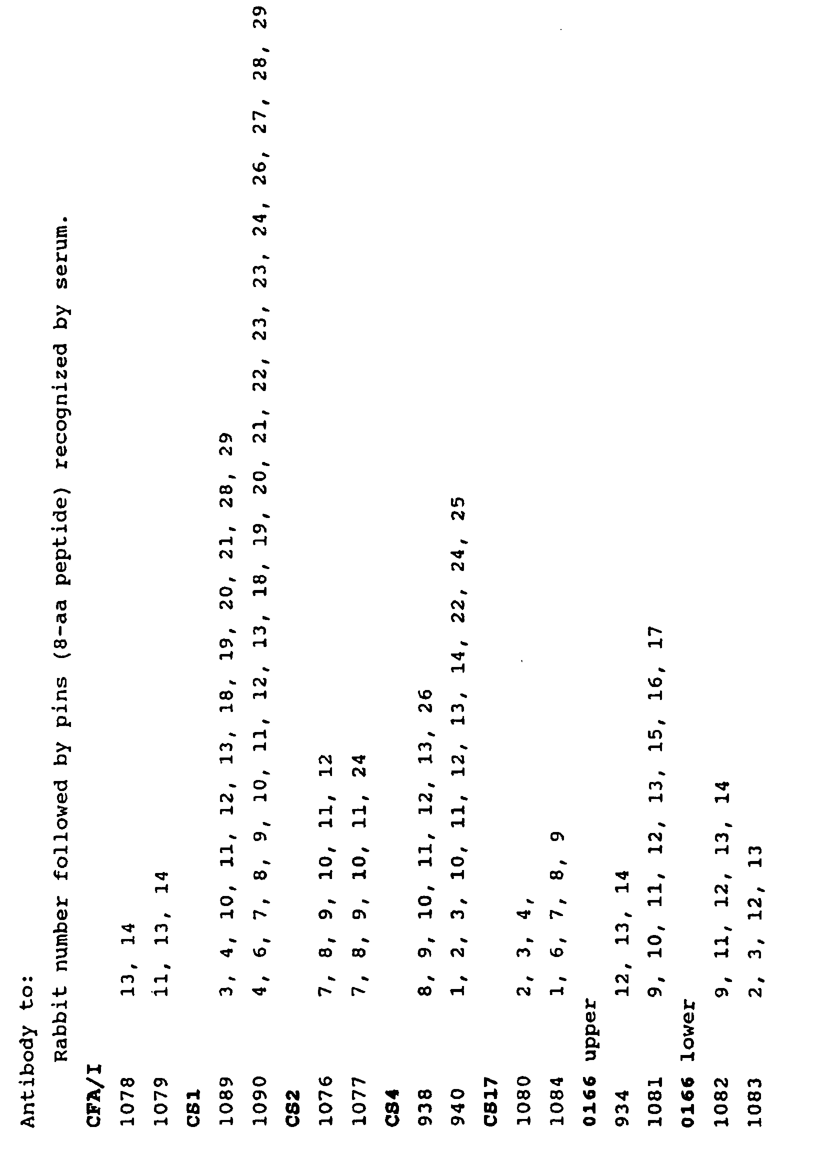

From the above data, units of the consensus peptide of Seq. #1 which could be expected to interact with nearly all antibodies arising in response to the natural organisms were identified. Such a peptide encompasses all the amino acids of Seq #12 through Seq #20., namely: TVTASVDPTIDLLQAD (Seq. #3). The sequence may be shortened somewhat with deletion of any or all of the first three amino acids and the last amino acid, but should contain the amino sequences ASVDPTIDLLQA (Seq. #2) for purposes of retaining activity against the target class of organisms. Peptides containing these sequences should react with most antibodies of the natural organisms producing CS4-CFA/I family of proteins and may be used to determine whether an individual animal has antibodies to ETEC E. coli. These sequences, as well as the larger consensus peptide and the other 8-mer peptides disclosed herein may be used to elicit antibodies to the natural organisms producing CS4-CFA/I family proteins.

The peptides of the invention are useful for immunization to raise antibodies to the organisms producing the CS4-CFA/I family of proteins. Particularly preferred sequences are those containing sequences 2, 3, 33 and 36, since these epitopes bind to the effective antibodies. For purposes of immunization, it is preferred that the peptides containing these sequences from these preferred sequences contain at least 16 amino acids. The peptides of the invention may be administered in pharmaceutically acceptable carriers for administration by usual means known in the art, including subcutaneously, intradramally, orally or nasally. Adjuvents known in the art may be used in such carriers. The immunogenic peptides may be administered as a primary dose with second and third dosings used a boosters, in accord with the teachings herein.

The antibodies raised to the peptides are useful for identifying members of the CS4-CFA/I family in cultures. Assay kits containing the antibodies may be prepared and may contain, in addition, agents for tagging for facilitated identification of the antibody/antigen complex. Such tags include radioactive isotopes, fluorescing agents and colorometric indicators. Such agents may be attached to solid supports. For example, an ELISA test kit system may be used to identify the antibody/antigen complex.

Compositions containing the antibodies raised in accord with the teachings herein may be prepared using a carrier appropriate for addition to a growth media. Saline and other buffered solutions known in the art are appropriate as carriers for the antibodies.

Antibodies raised to the sequences of the invention may be prepared in pharmaceutically acceptable carrier solutions and may be administered to the infected area to agglutinate the bacteria bearing CS4-CFA/I proteins. Administration would provide means for the compositions to contact the organisms. For example, the compositions could be administred orally in capsules which protect the antibody from distruction in the stomach and duodenum. The compositions are appropriate for use both for short-term prophylaxis and for treatment of ETEC E. coli infections by administration of an ETEC E. coli agglutinating effective amount of the pharmaceutical composition.

For use in vaccine compositions sequences containing at least one peptide of at least 8 amino acids but no more than 30 amino acids having sequences of a concensus peptide of Sequence #1 or #2, said peptides having sequences chosen from:
EKNITVTA (Seq. #8), KNITVTAS (Seq. #9), NITVTASV (Seq #10), ITVTASVD (Seq. #11), TASVDPTI (Seq. #14), ASVDPTID (Seq. #15), SVDPTIDL (Seq. #16), VDPTIDLL (Seq. #17), DPTIDLLQ (Seq. #18), PTIDLLQA (Seq. #19), SALPSAVA (Seq. #29), ALPSAVAL (Seq. #30), LPSAVALT (Seq. # 31), PSAVALTY (Seq. #32), SAVALTYS (Seq. #33), AVALTYSP (Seq. #34),and VALTYSPA (Seq. #35) PSAVALTYSP (Seq. #36), TVTASVDPTIDLLQAD (Seq. #3), and ASVDPTIDLLQA (Seq. #2) may be used. It is preferred that such peptides have at least 16 amino acids. The compositions for use as immunogens may also contain adjuvents used in the art.

### NUMBERED PARAGRAPHS:

1. A peptide of no more than 20 amino acids containing at least one of sequences 33, 36, 2 or 3.
2. A composition of matter containing at least one peptide of at least 16 to 30 amino acids having sequences chosen from: EKNITVTA (Seq. #8), KNITVTAS (Seq. #9), NITVTASV (Seq. #10), ITVTASVD (Seq. #11), TASVDPTI (Seq. #14), ASVDPTID (Seq. #15), SVDPTIDL (Seq. #16), VDPTIDLL (Seq. #17), DPTIDLLQ (Seq. #18), PTIDLLQA (Seq. #19), SALPSAVA (Seq. #29), ALPSAVAL (Seq. #30), LPSAVALT (Seq. #31), PSAVALTY (Seq. #32), SAVALTYS (Seq. #33), AVALTYSP (Seq. #34), VALTYSPA (Seq. #35), PSAVALTYSP (Seq. #36), TVTASVDPTIDLLQAD (Seq. #3), and ASVDPTIDLLQA (Seq. #2) in a pharmaceutically acceptable carrier.
3. A composition of matter containing antibodies that bind to a site containing the sequence ASVDPTIDLLQA (Seq. #2).
4. A composition of paragraph 3 in a carrier appropriate for application to a bacteria-containing growth media.
5. A composition of paragraph 3 wherein the antibody has a tag to assist in identification of the antibody/CS4-CFA/I family protein complex.
6. A composition of paragraph 5 wherein the tag is a fluorescing agent.
7. A composition of paragraph 5 wherein the tag is a colorometric tag.
8. A assay for detecting presence of organisms bearing the CS4-CFA/I family proteins comprising the steps of: 1) contacting a culture of organisms with a composition of paragraph 3; 2) allowing time for the antibodies in the composition of claim 3 to interact with the microbial organisms; and 3) examining the culture after interaction of step 2 to determine whether an CS4-CFA/I family protein/antibody complex has been formed.
9. A composition comprising an antibody of paragraph 1 in a pharmaceutically acceptable carrier.
10. A composition of paragraph 8 wherein the carrier is saline.
11. A method of treating illness arising from infection with bacteria bearing CS4-CFA/I family of proteins by administration of a bacteria-agglutinating effective amount of the composition of paragraph 8.
12. A method of immunising a susceptible host against illness arising from infection with bacteria which produce the CS4-CFA/I family of proteins comprising administration of an immunising-effective amount of a composition of paragraph 2.
13. A method of paragraph 12 wherein the composition contains an adjuvant.

## Claims

1. A composition of matter containing at least one peptide of at least 16 to 30 amino acids having sequences chosen from: EKNITVTA (Seq. #8), KNITVTAS (Seq. #9), NITVTASV (Seq. #10), ITVTASVD (Seq. #11), TASVDPTI (Seq. #14), ASVDPTID (Seq. #15), SVDPTIDL (Seq. #16), VDPTIDLL (Seq. #17), DPTIDLLQ (Seq. #18), PTIDLLQA (Seq. #19), SALPSAVA (Seq. #29), ALPSAVAL (Seq. #30), LPSAVALT (Seq. #31), PSAVALTY (Seq. #32), AVALTYSP (Seq. #34) and VALTYSPA (Seq. #35) in a pharmaceutically acceptable carrier.

2. Use of a composition according to claim 1 in the manufacture of an immunogen for use in immunizing a susceptible host against illness arising from infection with bacteria which produce the CS4-CFA/I family of proteins.

3. Use according to claim 2 wherein the composition contains an adjuvant.
